# FASCICULE DE BREVET EUROPEEN

(11) **EP 4 181 871 B1**
(45) Date de publication et mention de la délivrance du brevet: **11.02.2026**
(21) Numéro de dépôt: 21755518.4
(22) Date de dépôt: 16.07.2021
(51) Int. Cl.: A61K 8/895, A61K 8/81, A61K 8/92, A61Q 1/06

(54) **COMPOSITION COSMÉTIQUE POUR LES LÈVRES**
KOSMETISCHE ZUSAMMENSETZUNG FÜR DIE LIPPEN
COSMETIC COMPOSITION FOR THE LIPS

(30) Priorité: 17.07.2020 FR 2007524
(43) Date de publication de la demande: 24.05.2023
(73) Titulaire: L V M H RECHERCHE, 45800 St. Jean de Braye (FR)
(72) Inventeur: TORESSANI, Gaelle, 45800 SAINT JEAN DE BRAYE (FR); FABRIS, Marine, 45800 SAINT JEAN DE BRAYE (FR); VILETTE, Caroline, 45800 SAINT JEAN DE BRAYE (FR)
(74) Mandataire: Regimbeau
(86) Numéro de dépôt international: PCT/FR2021/051333
(87) Numéro de publication internationale: WO 2022/013509

(56) Documents cités:
- WO-A1-2019/002311
- WO-A1-2019/180387
- WO-A2-2009/042832
- FR-A1- 2 922 104
- FR-A1- 2 924 936
- FR-A1- 2 968 983
- US-A1- 2005 265 943
- US-A1- 2009 092 567
- UNKNOWN &: "Technical Data Sheet DOWSIL(TM) EL-9241 DM Silicone Elastomer Blend", 1 January 2017 (2017-01-01), pages 1 - 3, XP055793070, Retrieved from the Internet <URL:https://www.dow.com/content/dam/dcc/documents/en-us/productdatasheet/27/27-15/27-1581-dowsil-el-9241-dm-silicone-elast.pdf?iframe=true> [retrieved on 20210407]

## Description

### DOMAINE DE L'INVENTION

La présente invention concerne une composition de soin et/ou de maquillage des lèvres.

### ETAT DE LA TECHNIQUE

Les produits de soin et/ou de maquillage des lèvres destinés à embellir, protéger et/ou colorer les lèvres sont largement répandus. On connaît notamment des produits pour les lèvres, sous forme liquide, semi-fluide ou solides (stick), pour un dépôt brillant ou un dépôt mat sur les lèvres et présentant, pour les produits de maquillage, de bonnes propriétés de tenue de la couleur. Pour apporter de la matité, on utilise généralement des huiles volatiles et des charges, mais la stabilité de la composition peut être impactée et le dépôt du film avoir un aspect desséchant sur les lèvres.

La demande WO2009/042832 décrit une composition de rouge à lèvres comprenant un élastomère de silicone sous forme de gel, une huile apolaire, une cire polaire et une cire apolaire et des pigments.

Il subsiste donc toujours le besoin de développer de nouveaux produits de soin et/ou de maquillage des lèvres, en particulier des produits solides, pour l'obtention d'un dépôt homogène, confortable, mat et non desséchant sur les lèvres. Les consommatrices recherchent également des propriétés de douceur à l'application, avec un effet 'velours'.

La présente invention permet de répondre à ces besoins. La Demanderesse a en effet développé une composition comprenant une teneur totale en matière active d'élastomère(s) d'organopolysiloxane supérieure ou égale à 7%, de préférence supérieure ou égale à 10% en poids du total de la composition, dans une phase huileuse contenant au moins une huile apolaire et une cire polaire. De façon inattendue, les inventeurs ont obtenu une composition présentant une très bonne stabilité malgré l'incompatibilité des composés siliconés (élastomères d'organopolysiloxane) en une teneur importante avec les composés non siliconés de la phase huileuse, et d'autre part l'incompatibilité de l'huile apolaire avec la cire polaire, avec les propriétés recherchées de matité, confort et hydratation à l'application avec un dépôt à aspect velouté.

### EXPOSE DE L'INVENTION

L'invention concerne, selon un premier aspect une composition cosmétique solide comprenant, dans un milieu physiologiquement acceptable au moins :
un élastomère d'organopolysiloxane véhiculé dans une première huile, et une poudre d'élastomère d'organosiloxane distincte de l'élastomère d'organosiloxane véhiculé dans une huile
une huile apolaire distincte de la première huile,
une cire polaire,
la teneur totale en matière active d'élastomère(s) d'organopolysiloane étant supérieure ou égale à 7%, de préférence supérieure ou égale à 10% en poids par rapport au poids total de ladite composition. et la teneur totale en cire(s) étant supérieure ou égale à 5% en poids par rapport au poids totale de la composition

L'invention porte également sur un procédé cosmétique de soin et/ou de maquillage des lèvres comprenant l'application sur les lèvres d'au moins une couche d'une composition selon l'invention.

### DESCRIPTION DETAILLEE DE L'INVENTION

Un premier objet de l'invention est donc une composition cosmétique solide selon la revendication 1 et telle que définie ci-dessus.

Selon un mode particulier et préféré, la composition de l'invention est une composition anhydre.

Par 'anhydre', on entend notamment que l'eau n'est de préférence pas ajoutée délibérément dans les compositions mais peut être présente à l'état de trace dans les différents composés utilisés dans les compositions. En particulier, la composition selon l'invention comprend moins de 4 % en poids d'eau, de préférence moins de 3 %, de préférence moins de 2 %, plus préférentiellement moins de 1 %, encore plus préférentiellement moins de 0,5 % en poids d'eau, par rapport au poids total de ladite composition, voire est totalement exempte d'eau.

La composition de l'invention est une composition solide.

Par 'solide', on entend une composition présentant, à une température de 20°C et à pression atmosphérique (760 mm de Hg), une dureté supérieure à 30 Nm⁻¹, de préférence supérieure à 40 Nm⁻¹.

La dureté peut être mesurée à 20°C par la méthode dite « du fil à couper le beurre », qui consiste à couper transversalement un bâton de produit, de préférence cylindrique de révolution, à l'aide d'un fil métallique rigide de diamètre 250 µm en déplaçant le fil relativement au stick à une vitesse de 60 mm/min. La dureté des échantillons est exprimée en Gramme force (Gf) et peut être mesurée au moyen d'un texturomètre de type Texturomètre TAXT Plus. De préférence, la valeur obtenue doit être comprise entre 140 et 190.

La composition cosmétique de l'invention comprend une phase grasse ou huileuse.

On entend par « phase huileuse » une huile ou un mélange d'huiles miscibles entre elles. Par « huile », on entend, au sens de l'invention, un corps gras, non soluble dans l'eau, liquide à 25°C et pression atmosphérique.

La composition de l'invention comprend au moins une première huile, qui sert de véhicule à l'élastomère d'organopolysiloxane et une seconde huile distincte de la première huile. Ces huiles sont décrites ci-apès.

### ELASTOMERE D'ORGANOPOLYSILOXANE

La composition de l'invention comprend un ou plusieurs élastomère(s) d'organopolysiloxane en une teneur totale en matière active d'élastomère(s) d'organopolysiloxane d'au moins 7%, de préférence d'au moins 10% en poids par rapport au poids total de ladite composition.

En particulier, la teneur totale en matière active d'élastomère(s) d'organopolysiloxane ira de 10% à 30%, en particulier de 10% à 20% en poids par rapport au poids total de ladite composition.

La composition de l'invention comprend au moins un élastomère d'organopolysiloxane véhiculé dans une huile et une poudre d'élastomère d'organopolysiloxane distincte de l'élastomère d'organopolysiloxane sus-décrit véhiculé dans une huile, ladite poudre d'élastomère d'organopolysiloxane étant de préférence enrobée de résine de silicone.

L'association de ces élastomères particuliers permet d'obtenir un dépôt mat, confortable (souplesse du dépôt, absence de sensation de dessèchement), ainsi qu'une sensation veloutée et douce.

Ces composés et leurs teneurs respectives sont décrites ci-après.

### ELASTOMERE D'ORGANOPOLYSILOXANE VEHICULE DANS UNE PREMIERE HUILE

Par "véhiculé" on entend au sens de l'invention que l'élastomère est apporté dans la composition sous une forme pré-dispersée dans au moins une première huile, en particulier sous la forme d'un mélange homogène de particules d'élastomère dispersées dans la première huile, stable pendant au moins 24 heures à 20°C. De préférence, cet élastomère se présente sous la forme d'un gel dans au moins une première huile.

Par "élastomère d'organopolysiloxane" ou "élastomère siliconé" on entend un organopolysiloxane souple, déformable, ayant des propriétés viscoélastiques et notamment la consistance d'une éponge ou d'une sphère souple. Son module d'élasticité est tel que ce matériau résiste à la déformation et possède une capacité limitée à l'extension et à la contraction. Ce matériau est capable de retrouver sa forme originelle suite à un étirement.

Il s'agit plus particulièrement d'un élastomère siliconé réticulé.

L'élastomère peut être choisi parmi les élastomères non émulsionnants ou émulsionnants. De préférence il s'agira d'un élastomère non émulsionnant.

Le terme "non émulsionnant" définit des élastomères d'organopolysiloxane ne contenant pas de chaîne hydrophile, et en particulier ne contenant pas de motifs polyoxyalkylène (notamment polyoxyéthylène ou polyoxypropylène), ni de motifs polyglycéryle.

Ainsi, l'élastomère d'organopolysiloxane peut être obtenu par :
- réaction d'addition réticulation de diorganopolysiloxane contenant au moins un hydrogène lié au silicium et de diorganopolysiloxane ayant des groupements à insaturation éthylénique liés au silicium, notamment en présence de catalyseur platine;
- ou par réaction de condensation réticulation déshydrogénation entre un diorganopolysiloxane à terminaisons hydroxyle et un diorganopolysiloxane contenant au moins un hydrogène lié au silicium, notamment en présence d'un organoétain ;
- ou par réaction de condensation réticulation d'un diorganopolysiloxane à terminaisons hydroxyle et d'un organopolysiloxane hydrolysable ;
- ou par réticulation thermique d'organopolysiloxane notamment en présence de catalyseur organopéroxyde ;
- ou par réticulation d'organopolysiloxane par radiations de haute énergie telles que rayons gamma rayons ultraviolet faisceau électronique.

De préférence, l'élastomère d'organopolysiloxane est obtenu par réaction d'addition réticulation (A) de diorganopolysiloxane contenant au moins deux hydrogènes liés chacun à un silicium, et (B) de diorganopolysiloxane ayant au moins deux groupements à insaturation éthylénique liés au silicium, notamment en présence (C) de catalyseur platine.

En particulier, l'élastomère d'organopolysiloxane peut être obtenu par réaction de diméthylpolysiloxane à terminaisons diméthylvinylsiloxy et de méthylhydrogéno-polysiloxane à terminaisons triméthylsiloxy, en présence de catalyseur platine.

Le composé (A) est le réactif de base pour la formation d'organopolysiloxane élastomère et la réticulation s'effectue par réaction d'addition du composé (A) avec le composé (B) en présence du catalyseur (C).

Le composé (A) est en particulier un organopolysiloxane ayant au moins deux atomes d'hydrogène liés à des atomes de silicium distincts dans chaque molécule.

Le composé (A) peut présenter toute structure moléculaire, notamment une structure chaîne linéaire ou chaîne ramifiée ou une structure cyclique

Le composé (A) peut avoir une viscosité à 25 °C allant de 1 à 50000 centistokes, notamment pour être bien miscible avec le composé (B). Les groupes organiques liés aux atomes de silicium du composé ( A) peuvent être des groupes alkyles tels que méthyle, éthyle, propyle, butyle, octyle ; des groupes alkyles substitués tels que 2-phényléthyl, 2- phénylpropyl, 3,3,3-trifluoropropyl ; des groupes aryles tels que phényle, tolyle, xylyle ; des groupes aryles substitués tels que phényléthyl ; et des groupes hydrocarbonés monovalents substitués tels qu'un groupe époxy, un groupe ester carboxylate, ou un groupe mercapto.

Le composé (A) peut ainsi être choisi parmi les méthylhydrogénopolysiloxanes à terminaisons triméthylsiloxy, les copolymères diméthylsiloxaneméthylhydrogénosiloxane à terminaisons triméthylsiloxy, les copolymères cycliques diméthylsiloxaneméthylhydrogénosiloxane.

Le composé (B) est avantageusement un diorganopolysiloxane ayant au moins deux groupes alkényles inférieurs (par exemple en C2-C4) ; le groupe alkényle inférieur peut être choisi parmi les groupes vinyle, allyle, et propényle. Ces groupements alkényles inférieurs peuvent être situés en toute position de la molécule organopolysiloxane mais sont de préférence situés aux extrémités de la molécule organopolysiloxane. L'organopolysiloxane (B) peut avoir une structure à chaîne ramifiée, à chaîne linéaire, cyclique ou en réseau mais la structure en chaîne linéaire est préférée. Le composé (B) peut avoir une viscosité allant de l'état liquide à l'état de gomme. De préférence, le composé (B) a une viscosité d'au moins 100 centistokes à 25 °C.

Outre les groupes alkényles précités, les autres groupes organiques liés aux atomes de silicium dans le composé (B) peuvent être des groupes alkyles tels que méthyle, éthyle, propyle, butyle ou octyle ; des groupes alkyles substitués tels que 2-phényléthyle, 2- phénylpropyle ou 3,3,3-trifluoropropyle ; des groupes aryles tels que phényl, tolyl ou xylyl ; des groupes aryles substitués tels que phényléthyle ; et des groupes hydrocarbonés monovalents substitués tels qu'un groupe époxy, un groupe ester carboxylate, ou un groupe mercapto.

Les organopolysiloxanes (B) peuvent être choisis parmi les rnéthylvinylpolysiloxanes, les copolymères méthylvinylsiloxane-diméthylsiloxane, les diméthylpolysiloxanes à terminaisons diméthylvinylsiloxy, les copolymères diméthylsiloxane-méthylphénylsiloxane à terminaisons diméthylvinylsiloxy, les copolymères diméthylsiloxane- diphénylsiloxanemétliylvinylsiloxane à terminaisons diméthylvinylsiloxy, les copolymères diméthylsiloxaneméthylvinylsiloxane à terminaisons trirnéthylsiloxy, les copolymères diméthylsiloxaneméthylphénylsiloxane-méthylvinylsiloxane à terminaisons trirnéthylsiloxy, les méthyl(3,3,3-trifluoropropyl)polysiloxane à terminaisons diméthylvinylsiloxy, et les copolymères diméthylsiloxane-méthyl(3,3,3-trifluoropropyl)siloxane à terminaisons diméthylvinylsiloxy.

En particulier, l'élastomère d'organopolysiloxane peut être obtenu par réaction de diméthylpropylisoxane à terminaisons diméthylvinylsiloxy et de méthylhydrogénopoly-siloxane à terminaisons trirnéthylslioxy, en présence de catalyseur platine. Selon une autre variante, le composé (B) peut être un composé hydrocarboné insaturé, ayant au moins deux groupes alkényles inférieurs (par exemple en C2-C4) ; le groupe alkényle inférieur peut être choisi parmi les groupes vinyle, allyle, et propényle. Ces groupements alkényles inférieurs peuvent être situés en toute position de la molécule mais sont de préférence situés aux extrémités. A titre d'exemple, on peut citer l'hexadiène, et en particulier le 1,5-hexadiène.

Avantageusement, la somme du nombre de groupements éthyléniques par molécule du composé (B) et du nombre d'atomes d'hydrogène liés à des atomes de silicium par molécule du composé (A) est d'au moins 5.

Il est avantageux que le composé (A) soit ajouté en une quantité telle que le rapport moléculaire entre la quantité totale d'atomes d'hydrogène liés à des atomes de silicium dans le composé (A) et la quantité totale de tous les groupements à insaturation éthylénique dans le composé (B) soit compris dans la gamme de 1,5/1 à 20/1.

Le composé (C) est le catalyseur de la réaction de réticulation, et est notamment l'acide chloroplatinique, les complexes acide chloroplatinique-oléfine, les complexes acide chloroplatinique-alkenylsiloxane, les complexes acide chloroplatinique-dicétone, le platine noir, et le platine sur support. Le catalyseur (C) est de préférence ajouté de 0,1 à 1000 parts en poids, mieux de 1 à 100 parts en poids, en tant que métal platine propre pour 1000 parts en poids de la quantité totale des composés (A) et (B).

Comme élastomères non-émulsionnants , on peut par exemple utiliser ceux vendus sous les dénominations DOWSIL Silicone Elastomer Blend tels que ceux véhiculés dans une huile non volatile vendus sous l'appellation « DOWSIL, 9041 » et « DOWSIL, EL -9241 DM » par la société Dow Chemical Company, ainsi que ceux véhiculés dans une huile volatile vendus sous l'appellation « DOWSIL EL-8040 ID », « DOWSIL EL-9140 DM », « DOWSIL EL-9240 DM », « DOWSIL EL- 9048 », « DOWSIL 9040 », « DOWSIL 9045 », « DOWSIL EB-9586 », « DOWSIL 9546 » par la société Dow Chemical Company.

On peut encore mentionner ceux vendus sous les dénominations, " KSG-15 "."KSG-1510", "USG-1G3", "USG-106", " KSG-16 ", "KSG-1610", " KSG-18A","KSG-19", "KSG-016F", KSG-41 A ", " K SG -42 A ", " KSG-43 ", " KSG-44 " "KSG-042Z" " SG-045Z", "KSG Q48Z" par la société Shin Etsu. D'autres élastomères non-émulsionnants véhiculés dans une huile non volatile ou faiblement volatile sont également vendus sous les dénominations "Gransil DM- 10", "Gransil DMAM", "Gransil DMG-20", "Gransil DMG-6", "Gransil PM" par la société Grant Industries; ou ceux véhiculés dans un mélange d'huiles vendus sous les appellations "Gransil OHS-5", "Gransil PS-5"par la société Grant industries; ou ceux véhiculés dans une huile volatile vendus sous les appellations "GI CD-10", "GI CD-11". "Gransil DMG- " Gransil DMG-3". "Gransil GCM-5", "Gransil GTS", "Gransil G VL", "Gransil GVL-HV", "Gransil IDS-5", "Gransil MLB", "Gransil PC-12", "Gransil PC-12P", "Gransil RPS", "Gransil RPS-D6", "GI CD-965", "Gransil DM-5", "Gransil DMCM-5", "Gransil DMDM-25", "Gransil DMDM-35", "Gransil DMID", "Gransil DMT3" "Gransil G AM" par la société Grant Industries.

La teneur en matière active d'élastomère(s) d'organopolysiloxane véhiculé(s) dans une première huile, pour les références commerciales les plus concentrées (fort taux de matière sèche) peut aller de 10% à 30% en poids, en particulier de 10% à 20% en poids par rapport au poids total de la composition.

Selon un mode particulier, notamment lorsque la composition comprend l'association d'un élastomère d'organopolysiloxane véhiculé dans une huile et d'une poudre d'élastomère d'organopolysiloxane distincte de l'élastomère sus-décrit , la composition selon l'invention comprend une teneur en élastomère(s) d'organopolysiloxane véhiculé(s) dans une première huile, dans une teneur en matière sèche (matière active) allant de 0,1% à 10%, en particulier 1% à 8%, de préférence de 2% à 6% en poids, par rapport au poids total de la composition. Par « première huile », on entend l'huile dans laquelle est véhiculé l'élastomère d'organopolysiloxane. Cette première huile est choisie parmi les huiles non volatiles et les huiles volatiles, de préférence les huiles non volatiles. Il s'agira notamment d'une huile siliconée.

Par « huile siliconée », on entend selon l'invention une huile comprenant au moins un atome de silicium, et notamment au moins un groupe Si-O.

A titre d'exemples, on peut citer les polydiméthylsiloxanes (PDMS) non volatiles, les polydiméthylsiloxanes comportant au moins un groupement alkyle ou alcoxy, en C₂₋₂₄, pendant et/ou en bout de chaîne siliconée. Comme polydiméthylsiloxanes, on peut citer notamment l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, et leurs mélanges. En particulier, la première huile est choisie parmi les polydiméthylsiloxanes linéaires de viscosité comprise entre 5 cSt et 100 cSt (centistokes); ou un de leurs mélanges.

Selon un mode particulier, la composition de l'invention comprend en outre une poudre d'élastomère d'organopolysiloxane, distincte de l'élastomère d'organopolysiloxane sus-décrit véhiculé dans la première huile.

### POUDRE D'ELASTOMERE D'ORGANOPOLYSILOXANE ENROBEE OU NON DE RESINE DE SILICONE

Ainsi la composition de l'invention comprend en outre une poudre d'élastomère d'organopolysiloxane distincte de l'élastomère d'organopolysiloxane sus-décrit véhiculé dans la première huile, ladite poudre d'élastomère d'organopolysiloxane étant enrobée ou non de résine de silicone. Avantageusement, la composition comprend en outre au moins une poudre d'élastomère d'organopolysiloxane enrobée de résine de silicone. Selon un mode particulier, la composition selon l'invention comprend une poudre d'élastomère d'organopolysiloxane non enrobée de résine de silicone. Comme élastomères sous forme de poudre, on peut utiliser ceux vendus sous les dénominations "DC9505", "DC 9506" par la société Dow Corning et de nom INCI Dimethicone/Vinyl Dimethicone Crosspolymer.

Selon un autre mode particulier et préféré, la composition selon l'invention comprend une poudre d'élastomère d'organopolysiloxane enrobée de résine de silicone, notamment de résine silsesquioxane, comme décrit par exemple dans le brevet US 5 538 793. De telles poudres d'élastomères sont vendues sous les dénominations « KSP-100 », « KSP-101 », « KSP-102 », « KSP-103 », « KSP-104 », « KSP-105 » par la société Shin Etsu, et ont pour nom INCI « vinyl dimethicone/methicone silsesquioxane Crosspolymer ».

De façon préférée, la poudre d'élastomère d'organopolysiloxane enrobée de résine de silicone est un composé de nom INCI « vinyl dimethicone/methicone silsesquioxane Crosspolymer ». De façon préférée, la composition selon l'invention comprend une poudre d'élastomère d'organopolysiloxane enrobée de résine de silicone, notamment de résine silsesquioxane, en une teneur allant de de 1% à 20%, en particulier de 5% à 15%, de préférence de 7% à 11% en poids par rapport au poids total de la composition

Selon un mode particulier et préféré, la composition comprend ainsi un élastomère d'organopolysiloxane véhiculé dans une huile non volatile associé à au moins une poudre d'élastomère d'organopolysiloxane, avantageusement enrobée d'une résine de silicone. Ainsi, selon un mode particulier, la teneur en matière active d'élastomère d'organopolysiloxane véhiculé dans une huile va de 0,1% à 10%, en particulier 1% à 8%, de préférence de 2% à 6% en poids par rapport au poids total de la composition et la teneur en poudre d'élastomère d'organopolysiloxane va de 1% à 20%, en particulier 5% à 15%, de préférence 7% à 11% en poids par rapport au poids total de la composition.

### HUILE APOLAIRE

La composition cosmétique selon l'invention comprend au moins une « seconde huile », distincte de la première huile mentionnée ci- dessus. Cette deuxième huile est choisie parmi les huiles non volatiles ou les huiles volatiles, ou leur mélange, de préférence les huiles non volatiles.

La deuxième huile selon l'invention est une « huile apolaire » autrement nommée « huile non polaire ».

Par « huile apolaire » au sens de la présente invention, on entend une huile dont le paramètre de solubilité à 25 °C, δa, est égal à 0 (J/cm³)^{1/2}. La définition et le calcul des paramètres de solubilité dans l'espace de solubilité tridimensionnel de HANSEN sont décrits dans l'article de C. M. HANSEN : "The three dimensionnal solubility parameters" J. Paint Technol. 39, 105 (1967).

L'huile apolaire peut être une huile siliconée ou une huile hydrocarbonée.

En particulier, il s'agira d'une l'huile hydrocarbonée apolaire.

L'huile hydrocarbonée apolaire peut être choisie parmi les hydrocarbures linéaires ou ramifiés, d'origine minérale ou synthétique tels que : l'huile de paraffine ou ses dérivés, le squalane, l'isoeicosane, l'huile de naphtalène, les polybutylènes, les polyisobutylènes hydrogénés, les copolymères décène/butène, les copolymères polybutène/polyisobutène, les polydécènes et les polydécènes hydrogénés, et leurs mélanges.

De préférence, la composition de l'invention comprend au moins une huile apolaire choisie parmi les polybutylènes, les polyisobutylènes hydrogénés, les copolymères décène/butène, les copolymères polybutène/polyisobutène, les polydécènes et les polydécènes hydrogénés, de préférence encore les polydécènes hydrogénés.

La teneur en huile apolaire(s) selon l'invention ira généralement de 15% à 45%, en particulier de 20% à 40%, en poids par rapport au poids total de ladite composition.

La composition de l'invention peut comprendre en outre d'autres huiles additionnelles choisies parmi les huiles hydrocarbonées, les huiles siliconées, et leurs mélanges.

Selon un mode particulier, la composition comprend en outre au moins une huile hydrocarbonée polaire.

Par « huile polaire » au sens de la présente invention, on entend une huile dont le paramètre de solubilité à 25 °C, δa, est différent de 0 (J/cm³)^{1/2}.

En particulier, la ou les huiles hydrocarbonées polaires comprennent au moins une fonction alcool (il s'agit alors d'une « huile alcool ») ou au moins une fonction ester (il s'agit alors d'une « huile ester »).

Parmi les « huiles alcools », on peut citer notamment les alcools en C10-C26, plus particulièrement en C10-C24, et de préférence en C12-C22, saturés ou non, ramifiés ou non, plus particulièrement les monoalcools. Plus particulièrement, les alcools en C10-C26 sont des monoalcools gras, de préférence ramifiés lorsqu'ils comprennent au moins 16 atomes de carbone. A titre d'exemples d'alcools gras pouvant être utilisés selon l'invention, on peut citer les alcools gras linéaires ou ramifiés, d'origine synthétique, ou encore naturelle. A titre d'exemples particuliers d'alcools gras utilisables à titre préféré, on peut notamment citer l'alcool laurique, isostéarylique, oléique, le 2-butyloctanol, le 2-undécyl pentadécanol, l'alcool 2-hexyldécylique, l'alcool isocétylique, l'octyldodécanol et leurs mélanges. Selon un mode de réalisation avantageux de l'invention, l'alcool gras est l'octyldodécanol.

Ainsi selon un mode particulier, la composition de l'invention comprendra en outre de l'octyldodecanol.

Selon un mode particulier, la composition de l'invention comprend en outre une huile polaire.

Parmi les « huiles esters », on peut notamment citer :
- les esters d'acide gras, en particulier de 4 à 22 atomes de carbone,
- les esters de synthèse comme les huiles de formule R₁COOR₂ dans laquelle R₁ représente le reste d'un acide gras linéaire ou ramifié comportant de 4 à 40 atomes de carbone et R₂ représente une chaîne hydrocarbonée notamment ramifiée contenant de 4 à 40 atomes de carbone à condition que R₁+R₂ soit ≥16, comme par exemple l'isononanoate d'isononyle, le palmitate d'éthyle 2-hexyle, le néopentanoate d'octyledodécyle, le stéarate d'octyl-2 dodécyle, l'isostéarate d'isostéaryle, le benzoate d'octyl-2 dodécyle, le myristate d'isopropyle, le palmitate d'isopropyle, le stéarate de butyle, le laurate d'hexyle, le palmitate de 2-éthyl-hexyle, le laurate de 2-hexyl-décyle, le palmitate de 2-octyl-décyle, le myristate de 2-octyldodécyle, le succinate de 2-diéthyl- hexyle;
- les esters d'acides gras linéaires ayant un nombre total de carbone allant de 35 à 70 ;
- les esters hydroxylés, de préférence ayant un nombre total de carbone allant de 35 à 70, comme le triisostéarate de polyglycérol-2, le lactate d'isostéaryle, l'hydroxystéarate d'octyldodécyle, le malate de diisostéaryle, le stéarate de glycérine ; le diisononanoate de diéthylèneglycol ;
- les esters d'acides aromatiques et d'alcools comprenant 4 à 22 atomes
- les esters d'alcool gras ou d'acides gras ramifiés en C24-C28
- les polyesters résultant de l'estérification d'au moins un triglycéride d'acide(s) carboxylique(s) hydroxylé(s) par un acide monocarboxylique aliphatique et par un acide dicarboxylique aliphatique, éventuellement insaturé,
et leurs mélanges.

Une composition selon l'invention peut ainsi comprendre, selon un mode particulier, une teneur en huile(s) polaire(s) allant de 1% à 15 %, par exemple de 2% à 10 % en poids par rapport au poids total de la composition.

La quantité totale d'huiles présentes dans la composition selon l'invention est comprise entre 40% et 75% en poids, de préférence entre 50% et 70% par rapport au poids total de la composition.

### CIRE POLAIRE

La composition selon l'invention comprend au moins une cire polaire.

Par « cire », au sens de la présente invention, entend désigner un composé solide à 25 °C qui présente un changement d'état solide/liquide réversible et une température de fusion supérieure à 30 °C, de préférence supérieure à 45°C.

En particulier, la cire polaire est choisie dans le groupe comprenant la cire d'abeille, la cire de camauba, la cire de candelilla, la cire de coton, la cire de son de riz, la cire de tournesol, la cire de baie, la cire d'insecte de chine, la cire de montan, la lanoline et ses dérivés alcools, acétylés, estérifiés, polyéthoxylés, la cire de kapok, la cire de canne à sucre, le laurate d'hexyle, la cire de jojoba, la cire shellac, l'éther de cholestérol polyéthoxylé, les cires d'abeille synthétiques commercialisées par Koster Keunen sous la dénomination commerciale Kester Wax K82H, ou un de leurs mélanges.

Selon un mode particulier, la composition de l'invention comprend une ou plusieurs cire(s) polaire(s) choisie(s) dans le groupe constitué de la cire de candelilla, la cire d'abeille, la cire de camauba, la cire de son de riz, la cire de tournesol, la cire de jojoba, et leurs mélanges, de préférence la cire de candelilla.

Selon un mode préféré, la composition de l'invention comprend au moins comme cire polaire, une cire de candelilla.

La teneur en cire(s) polaire(s) dans la composition de l'invention ira généralement de 0,5% à 10%, de préférence de 1% à 5% en poids par rapport au poids total de ladite composition.

La composition peut comprendre en outre une cire apolaire.

Par cire apolaire, on entend une cire hydrocarbonée et/ou une cire siliconée.

On entend par « cire apolaire hydrocarbonée », une cire comprenant uniquement des atomes de carbone et d'hydrogène et ne comprenant pas d'hétéroatomes tels que l'oxygène, l'azote, le silicium ou le phosphore.

On peut utiliser notamment comme cire apolaire, les cires microcristallines, les paraffines, l'ozokérite, les cires de polyéthylène, et leurs mélanges, de préférence les cires de polyéthylène.

On entend par « cire apolaire siliconée », une cire comprenant un hétéroatome de silicium.

Des exemples de cires apolaires siliconées comprennent la C20-24 alkyl diméthicone, commercialisée par Siltech sous la dénomination Silwax D2024, la C24-28 alkyle diméthicone, commercialisée par Evonik Industries AG sous la dénomination Abil Wax, ou un de leurs mélanges.

La teneur en cire(s) apolaire(s) dans la composition de l'invention pourra aller de 0,5 à 12%, en particulier de 2% à 10%, voire de 4% à 8% en poids par rapport au poids total de ladite composition.

Selon un mode particulier, la composition de l'invention comprendra, outre la cire polaire, également une cire apolaire, de préférence choisie parmi les cires de polyéthylène.

Ainsi selon un mode particulier, la composition de l'invention comprend au moins une cire de candelilla et une cire de polyéthylène.

En particulier, une composition selon l'invention peut comprendre une teneur totale en cires allant de 5 % à 20 %, par exemple de 6 % à 15 % en poids, de préférence de 7 % à 12 % en poids, par rapport au poids total de la composition.

La teneur totale en cire(s) dans la composition de l'invention est supérieure ou égale à 5% en poids par rapport au poids total de ladite composition.

### INGREDIENTS ADDITIONNELS

La composition de l'invention peut comprendre en outre un corps gras pâteux.

### Corps gras pâteux

Par « composé gras pâteux » ou « composé pâteux » ou « corps gras pâteux », on désigne un composé gras non cristallin comprenant à une température de 25°C, une fraction liquide et une fraction solide.

Le composé pâteux est par exemple choisi dans le groupe constitué de la lanoline et de ses dérivés, les composés siliconés polymères, les copolymères de (méth)acrylates d'alkyles ayant de préférence un groupement alkyle en C8-C30, les oligomères homo et copolymères d'esters vinyliques ayant des groupements alkyles en C8-C30, les oligomères homo et copolymères de vinyl-éthers ayant des groupements alkyles en C8-C30, les polyéthers liposolubles résultant de la polyéthérification entre un ou plusieurs diols en C2-C50, les copolymères d'éthylène-oxyde et/ou de propylène-oxyde avec des alkylènes-oxydes à longue chaîne en C6-C30, les esters de diglycérol, le propionate d'arachidyle, les esters de phytostérol, les polyesters non réticulés résultant de la polycondensation entre un diacide ou un polyacide carboxylique linéaire ou ramifié en C4-C50 et un diol ou un polyol, l'ester résultant de la réaction d'estérification de l'huile de ricin hydrogénée avec l'acide isostéarique tel qu'un mono- di- ou tri-isostéarate d'huile de ricin hydrogénée, un mélange de stérols de soja et de pentaérythritol oxyéthyléné (5 OE) oxypropyléné (5 OP), les triglycérides d'acides gras et leurs dérivés, le beurre de karité, le beurre de cacao, l'huile ou beurre de mangue, et leurs mélanges.

### Charges

La composition peut comprendre en outre au moins une charge additionnelle distincte de la poudre d'élastomère de silicone.

Au sens de l'invention, par « charges » il faut entendre des particules de toute forme, incolores ou blanches, de nature minérale ou organique, naturelle ou synthétique, qui se présentent sous une forme insoluble et dispersée dans le milieu de la composition. Ces charges servent notamment à modifier la rhéologie ou la texture de la composition et/ou conférer un effet matifiant. Les charges peuvent être minérales ou organiques de toute forme : plaquettaires, sphériques ou oblongues.

Les « charges » sont choisies notamment parmi les silices, les micas, d'origine naturelle ou synthétique, le kaolin, les oxydes de zinc et de titane ; le carbonate de calcium, le carbonate et l'hydrocarbonate de magnésium ; le stéarate de zinc, de magnésium ou de lithium, le laurate de zinc, le myristate de magnésium ; les poudres de polymères synthétiques, tels que le polyéthylène, les polyesters, les polyamides (par exemple le nylon) ; des poudres d'acides polyacrylique ou polyméthacrylique,; les poudres minérales telles que la silice sphérique ; les dioxydes de titane sphériques; les billes de verre et de céramique ; des poudres de matériaux organiques d'origine naturelle comme les amidons de maïs, de blé, de riz, réticulées ou non, et leurs mélanges.

Selon un mode particulier, on utilisera de la silice, de la poudre de cellulose, ou leur mélange.

Une composition selon l'invention peut comprendre une teneur en charge(s) allant de 0,5% à 10 %, par exemple de 1% à 8 % en poids, de préférence de 2% à 7 % en poids, par rapport au poids total de la composition.

### Matières colorantes

Une composition conforme à la présente comprend avantageusement, pour maquiller les lèvres, au moins une « matière colorante » pouvant être choisie parmi les matières colorantes hydrosolubles ou non, liposolubles ou non, organiques ou inorganiques, les matériaux à effet optique, et leurs mélanges.

On entend par matière colorante au sens de la présente invention, un composé susceptible de produire un effet optique coloré lorsqu'il est formulé en quantité suffisante dans un milieu cosmétique approprié.

Selon un mode particulier, la ou les matières colorantes sont notamment choisies parmi des pigments minéraux et/ou organiques, des pigments composites (à base de matériaux minéraux et/ou organiques), des colorants, des nacres ou pigments nacrés, et leurs mélanges.

Par 'colorants', on entend des colorants utilisés classiquement dans le domaine cosmétique, distincts des colorants alimentaires utilisés dans les produits alimentaires.

Selon un mode particulier, la composition de l'invention comprend au moins une matière colorante choisie parmi des pigments minéraux et/ou organiques, des pigments composites (à base de matériaux minéraux et/ou organiques), des nacres ou pigments nacrés, et leurs mélanges.

### Pigments minéraux et organiques

Par « pigments » on entend des particules blanches ou colorées, minérales ou organiques, insolubles dans une solution aqueuse, destinées à colorer et/ou opacifier le dépôt résultant. On peut citer les pigments minéraux, les pigments organiques, et les pigments composites (c'est-à-dire des pigments à base de matériaux minéraux et/ou organiques).

Parmi les « pigments minéraux », on peut citer, à titre d'exemples le dioxyde de titane ; les oxydes de fer noir, jaune, rouge et brun et le violet de manganèse.

Parmi les « pigments organiques », on peut citer, par exemple, les pigments D & C red n° 19; D & C red n° 9; D & C Red n° 22 ; D & C Red n° 21 ; D & C Red n° 28 ; D & C Yellow n° 6 ; D & C orange n° 4 ; D & C orange n° 5 ; D & C Red n° 27; D & C red n° 13; D & C Red n° 7 ; D & C Red n° 6 ; D & C Yellow n° 5; D & C Red n° 36 ;; D & C Red n° 33 ; D & C orange n° 10; D & C yellow n° 6 ; ; D & C Red n° 30 ; D &C red n° 3 ; D &C Blue 1 ; le noir de carbone et les laques à base de carmin de cochenille.

### Colorants

Parmi les « colorants », on pourra citer Yellow 5, Yellow 6, Blue 1, Green 5, Green 3, Green 6, Orange 4, Red 4, Red 21, Red 22, Red 27, Red 28, Red 33, Red 40, le carminé de cochenille (CI 15850, CI 75470). Les colorants liposolubles sont par exemple le rouge Soudan, le D&C Red 17, le D&C Green 6, le bêta-carotène, le brun Soudan, le D&C Yellow 11, le D&C Violet 2, le D&C Orange 5, le jaune quinoléine, le rocou.

En particulier, la teneur totale en matières colorantes dans la composition pourra aller de 1% à 15% en poids, en particulier de 2% à 10% en poids, de préférence encore de 3% à 8% en poids par rapport au poids total de la composition.

### GALENIQUE

La composition de l'invention est une composition de soin et/ou de maquillage des matières kératiniques, en particulier une composition de maquillage et/ou de soin des lèvres.

La composition de l'invention est généralement une composition solide, telle que définie précédemment.

Comme composition de maquillage pour les lèvres, on peut citer notamment un stick pour les lèvres, un crayon contour des lèvres, ou un baume à lèvres.

Selon un mode particulier et préféré, il s'agira d'une composition solide de maquillage des lèvres, en particulier un stick ou rouge à lèvres.

### PROCEDE COSMETIQUE

L'invention porte également sur un procédé cosmétique de soin et/ou de maquillage des lèvres, comprenant l'application d'une composition selon l'invention.

La composition appliquée sur les lèvres est de préférence une composition solide de maquillage des lèvres, en particulier un stick ou rouge à lèvres.

Le procédé selon l'invention est en particulier un procédé de maquillage des lèvres, destiné déposer sur lesdites lèvres un film confortable, mat et non desséchant sur les lèvres, avec une bonne tenue de la couleur.

L'invention va être illustrée par les exemples non limitatifs suivants. Les pourcentages sont exprimés en pourcentage en poids par rapport au poids total de la composition sauf indication contraire.

### EXEMPLES

### Exemple 1 : Effet de la présence d'une cire polaire dans la composition de l'invention

Deux formules comparatives ont été testées, l'une avec la présence d'une cire polaire (invention) et l'autre sans la présence de cire polaire (comparatif). La cire polaire utilisée est la cire de candelilla.

On a évalué la mise en oeuvre desdites compositions, ainsi que les propriétés de stabilité de la composition et de sensorialité.

Les résultats sont présentés dans le tableau suivant :

**[Table 1]**

| Ingrédients | | Composition hors invention (sans cire polaire) | Composition selon l'invention (avec cire polaire) |
|---|---|---|---|
| Elastomère de silicone véhiculé dans une huile (DOWSIL EL 9241 DM) | | 25 | 25 |
| Huile de silicone (DIMETHICONE 100 CS) | | 7,8 | 7,8 |
| Lécithine EMULMETIK 300 IP | | 0,5 | 0,5 |
| UNIPURE RED LC | | 0,9875 | 0,9875 |
| C 339001 SUN PURO | | 2,48 | 2,48 |
| UNIPURE RED LC | | 0,77 | 0,77 |
| SUNCROMA FDC YELLOW 6 AL LAKE | | 4,8 | 4,8 |
| SUNCROMA FDC YELLOW 5 AL LAKE | | 2,6 | 2,6 |
| UNIPURE RED LC | | 5,55 | 5,55 |
| Polydécène hydrogéné DEKANEX 2006 FG (huile apolaire) | | 28,0325 | 28,0325 |
| Cire de polyéthylène PERFORMALENE 500 (cire apolaire) | | 7,45 | 5,2 |
| Cire de candelilla CANDELILLA WAX SR 3 (cire polaire) | | - | 2,5 |
| Silice SUNSPHERE H33 | | 3 | 3 |
| Silice SILICA SHELLS | | 2 | 2 |
| Poudre d'élastomère de silicone enrobée de résine siliconée (KSP-100) | | 3 | 3 |
| Carbonate de calcium | | 0,05 | 0,05 |
| Beurre de karité | | 1,5 | 1,5 |
| Stabilité | | Présence de trous dans les sticks, impossible d'avoir un stick complet | OK |
| Sensorialité | | Difficile à couler un stick correct | OK |

Les compositions sont préparées selon le protocole suivant :
- l'élastomère de silicone véhiculé dans une huile (Dowsil 9241 EL) et la Dimethicone 100cs sont mélangés sous agitation à 95°C ; lorsque le mélange est homogène, sont incorporées les cires de polyéthylène et de candelilla ;
- la pâte pigmentaire comprenant les pigments, le polydécène hydrogéné et la lécithine est ajoutée au mélange préparé ci-dessus lorsque les cires sont fondues, puis on saupoudre les poudres (charges : silica shells, sunsphère H33 et KSP 100) sous agitation ;
- le mélange à une température supérieure à 95°C est coulé dans un moule.

Les rouges à lèvres sont évaluées sur plusieurs critères :
- Caractéristiques organoleptiques : sensorialité, couleur : les compositions sont testées sensoriellement pour leurs propriétés de confort, matité et couvrance.
- Caractéristiques physico chimiques : dureté, test de casse : les mesures physico chimiques permettent de s'assurer de la mise en forme possible de la composition de l'invention, de la tenue du stick lors de l'application et de sa casse. En pratique, les raisins sont placés à 20°C pendant 24h. Ils sont ensuite coupés à 1cm de la cupule à l'aide d'un fil pour mesurer la dureté du stick. L'opération est effectuée trois fois afin d'effectuer une moyenne. La dureté nous permet de prévoir le comportement du stick au démoulage, en casse et à l'application. Pour la mesure de la dureté est utilisé un texturomètre, le TAXT Plus et le logiciel Exponent. La valeur obtenue, exprimée en Gramme force (Gf), doit être comprise entre 140 et 190.
- Stabilité : Les formules sont suivies dans différentes étuves comme détaillé ci-après pour reproduire la vie d'un produit. Ces tests de vieillissement accéléré nous permettent d'assurer que le produit est stable dans le temps. Nous pouvons ainsi confirmer la durabilité du produit dans des conditions normales d'utilisation et de stockage. En pratique, on observe la stabilité des sticks après passage dans différentes étuves : humidité, alternance, 4°C et 45°C. Les pièces sont mises en stabilités 24h après leur fabrication. On observe les échantillons après un temps défini à chaque étuve 24h, 2 semaines, 1 mois, 3 mois. Il est observé l'absence de dérive couleur, l'absence de goutte, d'exsudation et de modifications de l'aspect du raisin : brillance, matité.

Les résultats montrent que la présence de la cire polaire dans la composition de l'invention permet d'obtenir une composition stable et qui présente les caractéristiques organoleptiques et physico-chimiques recherchées, comparativement à la même composition sans cire polaire.

Cette composition permet d'obtenir un stick mat avec une forte couvrance, ayant une structure assez « rigide » pour avoir une structure non guidée et confortable (pas de tiraillement dans le temps). Par 'structure non guidée', on entend une formulation pour une application avec le raisin (tube de rouge à lèvres) se présentant hors du mécanisme.

Exemple 2 : Effet de la présence d'une cire polaire dans une composition de l'invention On compare deux formules, l'une avec la présence d'une cire polaire (invention) et l'autre sans la présence de cire polaire (comparatif). La cire polaire utilisée est la cire de candelilla.

**[Table 2]**

| Ingrédients | Composition hors invention (sans cire polaire) | Composition selon l'invention (avec cire polaire) |
|---|---|---|
| Elastomère de silicone véhiculé dans une huile (DOWSIL EL 9241 DM) | 25 | 25 |
| Huile de silicone (DIMETHICONE 100 CS) | 7,8 | 7,8 |
| Lécithine EMULMETIK 300 IP | 0,5 | 0,5 |
| UNIPURE RED LC | 0,9875 | 0,9875 |
| C 339001 SUN PURO | 2,48 | 2,48 |
| UNIPURE RED LC | 0,77 | 0,77 |
| SUNCROMA FDC YELLOW 6 AL LAKE | 4,8 | 4,8 |
| SUNCROMA FDC YELLOW 5 AL LAKE | 2,6 | 2,6 |
| UNIPURE RED LC | 5,55 | 5,55 |
| Polydécène hydrogéné DEKANEX 2006 FG (huile apolaire) | qsp 100 | qsp 100 |
| Cire de polyéthylène PERFORMALENE 500 (cire apolaire) | 7,45 | 5,2 |
| Cire de candelilla CANDELILLA WAX SR 3 (cire polaire) | - | 2,5 |
| Silice SUNSPHERE H33 | 3 | 3 |
| Silice SILICA SHELLS | 2 | 2 |
| Poudre d'élastomère de silicone réticulé (DIMETHICONE/VINYL DIMETHICONE CROSSPOLYMER - DOW CORNING 9506 POWDER) | 6,5 | 6,5 |
| Poudre d'élastomère de silicone enrobée de résine siliconée (KSP-100) | 3 | 3 |
| Carbonate de calcium | 0,05 | 0,05 |
| Beurre de karité | 1,5 | 1,5 |

On prépare les compositions selon le protocole suivant :
- l'élastomère de silicone véhiculé dans une huile (Dowsil 9241 EL) et la Dimethicone 100cs sont mélangés sous agitation à 95°C ; lorsque le mélange est homogène, sont incorporées les cires de polyéthylène et de candelilla ;
- la pâte pigmentaire comprenant les pigments, le polydécène hydrogéné et la lécithine est ajoutée au mélange préparé ci-dessus lorsque les cires sont fondues, puis on saupoudre les poudres (charges : silica shells, sunsphère H33, Dow Corning 9506 Powder et KSP 100) sous agitation ;
- le mélange à une température supérieure à 95°C est coulé dans un moule.

On évalue les rouges à lèvres sur plusieurs critères :
- Caractéristiques organoleptiques : sensorialité, couleur : on teste les compositions sensoriellement pour leurs propriétés de confort, matité et couvrance.
- Caractéristiques physico chimiques : dureté, test de casse : les mesures physico chimiques permettent de s'assurer de la mise en forme possible de la composition de l'invention, de la tenue du stick lors de l'application et de sa casse. En pratique, on place les raisins à 20°C pendant 24h. Ils sont ensuite coupés à 1cm de la cupule à l'aide d'un fil pour mesurer la dureté du stick. L'opération est effectuée trois fois afin d'effectuer une moyenne. La dureté nous permet de prévoir le comportement du stick au démoulage, en casse et à l'application. Pour la mesure de la dureté est utilisé un texturomètre, le TAXT Plus et le logiciel Exponent. La valeur obtenue, exprimée en Gramme force (Gf), doit être comprise entre 140 et 190.
- Stabilité : on suit les formules dans différentes étuves comme détaillé ci-après pour reproduire la vie d'un produit. Ces tests de vieillissement accéléré nous permettent d'assurer que le produit est stable dans le temps. Nous pouvons ainsi confirmer la durabilité du produit dans des conditions normales d'utilisation et de stockage. En pratique, on observe la stabilité des sticks après passage dans différentes étuves : humidité, alternance, 4°C et 45°C. Les pièces sont mises en stabilités 24h après leur fabrication. On observe les échantillons après un temps défini à chaque étuve 24h, 2 semaines, 1 mois, 3 mois. Il est observé l'absence de dérive couleur, l'absence de goutte, d'exsudation et de modifications de l'aspect du raisin : brillance, matité.

La composition de l'invention permet d'obtenir un stick mat avec une forte couvrance, ayant une structure assez « rigide » pour avoir une structure non guidée et confortable (pas de tiraillement dans le temps).

### Exemple 3 : Composition pour les lèvres à effet mat velours

**[Table 3]**

| Ingrédients | % |
|---|---|
| Polydécène hydrogéné | Qsp100 |
| Elastomère siliconé réticulé véhiculé dans une huile (DIMETHICONE/DIMETHICONE CROSSPOLYMER - DOWSIL EL 9241 DM) | 25.0 |
| Dimethicone 100cs | 8.0 |
| Cire de polyethylène | 5.5 |
| Poudre d'élastomère de silicone réticulé (DIMETHICONE/VINYL DIMETHICONE CROSSPOLYMER - DOW CORNING 9506 POWDER) | 3.0 |
| Octyldodecanol | 5.2 |
| Cire de candelilla | 3.25 |
| Poudre d'élastomère de silicone réticulé enrobée de résine de silicone (vinyl dimethicone/methicone silsesquioxane crosspolymer, KSP-100) | 3.0 |
| Pigments | 3.0 |
| Silice | 5.0 |
| Conservateurs | qs |

La composition est stable. Après application sur les lèvres, on obtient un dépôt confortable, mat et non desséchant, avec un aspect velouté.

### Exemple 4 : Composition pour les lèvres à effet mat velours

**[Table 4]**

| Ingrédients | % |
|---|---|
| Polydécène hydrogéné | Qsp100 |
| Elastomère siliconé réticulé véhiculé dans une huile (DIMETHICONE/DIMETHICONE CROSSPOLYMER - DOWSIL EL 9241 DM) | 25.0 |
| Dimethicone 100cs | 8.0 |
| Cire de polyethylène | 5.5 |
| Poudre d'élastomère de silicone réticulé (DIMETHICONE/VINYL DIMETHICONE CROSSPOLYMER - DOW CORNING 9506 POWDER) | 3.0 |
| Octyldodecanol | 5.2 |
| Cire de candelilla | 3.25 |
| Poudre d'élastomère de silicone réticulé enrobée de résine de silicone (vinyl dimethicone/methicone silsesquioxane crosspolymer, KSP-100) | 6,5 |
| Pigments | 3.0 |
| Silice | 5.0 |
| Conservateurs | qs |

La composition est stable. Après application sur les lèvres, on obtient un dépôt confortable, mat et non desséchant, avec un aspect velouté.

## Revendications

1. Composition cosmétique solide de préférence anhydre comprenant, dans un milieu physiologiquement acceptable au moins :
un élastomère d'organopolysiloxane véhiculé dans une première huile et une poudre d'élastomère d'organopolysiloxane distincte de l'élastomère d'organopolysiloxane véhiculé dans une huile
une huile apolaire distincte de la première huile,
une cire polaire,
la teneur totale en matière active d'élastomère(s) d'organopolysiloxane étant supérieure ou égale à 7%, de préférence supérieure ou égale à 10% en poids par rapport au poids total de ladite composition, et la teneur totale en cire(s) étant supérieure ou égale à 5% en poids par rapport au poids total de ladite composition.

2. Composition selon la revendication 1, **caractérisée en ce que** ladite poudre d'élastomère d'organopolysiloxane est enrobée de résine de silicone.

3. Composition cosmétique selon l'une quelconque des revendications 1 ou 2, **caractérisée en ce que** la teneur en matière active d'élastomère d'organopolysiloxane véhiculé dans une huile va de 0,1% à 10%, en particulier 1% à 8%, de préférence de 2% à 6% en poids par rapport au poids total de la composition et la teneur en poudre d'élastomère d'organopolysiloxane va de 1% à 20%, en particulier 5% à 15%, de préférence 7% à 11% en poids par rapport au poids total de la composition.

4. Composition cosmétique selon l'une quelconque des revendications précédentes, **caractérisée en ce que** la cire polaire est choisie dans le groupe constitué de la cire de candelilla, la cire d'abeille, la cire de carnauba, la cire de son de riz, la cire de tournesol, la cire de jojoba et leurs mélanges, de préférence la cire de candelilla.

5. Composition cosmétique selon l'une quelconque des revendications précédentes, **caractérisée en ce qu'**elle comprend en outre au moins une cire apolaire.

6. Composition cosmétique selon l'une quelconque des revendications précédentes, **caractérisée en ce qu'**elle comprend en outre une huile polaire.

7. Composition cosmétique selon l'une quelconque des revendications précédentes, **caractérisée en ce qu'**elle comprend en outre des matières colorantes, de préférence des pigments.

8. Composition cosmétique selon l'une quelconque des revendications précédentes, **caractérisée en ce qu'**il s'agit d'un produit de soin et/ou de maquillage pour les lèvres, en particulier un stick pour les lèvres, un crayon contour des lèvres, ou un baume à lèvres.

9. Procédé cosmétique de soin et/ou de maquillage des lèvres comprenant l'application sur les lèvres d'au moins une composition telle que définie dans l'une quelconque des revendications précédentes.

## Patentansprüche

1. Vorzugsweise wasserfreie feste kosmetische Zusammensetzung, die in einem physiologisch akzeptablen Medium mindestens umfasst:
ein Organopolysiloxan-Elastomer, das in einem ersten Öl transportiert wird und ein Organopolysiloxan-Elastomer-Pulver, das von dem Organopolysiloxan-Elastomer, das in einem Öl transportiert wird, unterschiedlich ist,
ein apolares Öl, das von dem ersten Öl unterschiedlich ist,
ein polares Wachs,
wobei der Gesamtwirkstoffgehalt des/der Organopolysiloxan-Elastomers/e größer oder gleich 7 Gew.-%, vorzugsweise größer oder gleich 10 Gew.-%, bezogen auf das Gesamtgewicht der Zusammensetzung, beträgt und der Gesamtwachsgehalt größer oder gleich 5 Gew.-%, bezogen auf das Gesamtgewicht der Zusammensetzung, beträgt.

2. Zusammensetzung nach Anspruch 1, **dadurch gekennzeichnet, dass** das Organopolysiloxan-Elastomer-Pulver mit Silikonharz ummantelt ist.

3. Kosmetische Zusammensetzung nach einem der Ansprüche 1 oder 2, **dadurch gekennzeichnet, dass** der Wirkstoffgehalt des Organopolysiloxan-Elastomers, das in einem Öl transportiert wird, 0,1 bis 10 Gew.-%, insbesondere 1 bis 8 Gew.-%, vorzugsweise 2 bis 6 Gew.-%, bezogen auf das Gesamtgewicht der Zusammensetzung, beträgt und der Gehalt an Organopolysiloxan-Elastomer-Pulver 1 bis 20 Gew.-%, insbesondere 5 bis 15 Gew.-%, vorzugsweise 7 bis 11 Gew.-%, bezogen auf das Gesamtgewicht der Zusammensetzung, beträgt.

4. Kosmetische Zusammensetzung nach einem der vorstehenden Ansprüche, **dadurch gekennzeichnet, dass** das polare Wachs aus der Gruppe ausgewählt ist, die aus Candelillawachs, Bienenwachs, Carnaubawachs, Reiskleiewachs, Sonnenblumenwachs, Jojoba-Wachs und deren Mischungen besteht, vorzugsweise Candelillawachs.

5. Kosmetische Zusammensetzung nach einem der vorstehenden Ansprüche, **dadurch gekennzeichnet, dass** sie ferner mindestens ein apolares Wachs umfasst.

6. Kosmetische Zusammensetzung nach einem der vorstehenden Ansprüche, **dadurch gekennzeichnet, dass** sie ferner ein polares Öl umfasst.

7. Kosmetische Zusammensetzung nach einem der vorstehenden Ansprüche, **dadurch gekennzeichnet, dass** sie ferner Farbstoffe, vorzugsweise Pigmente, umfasst.

8. Kosmetische Zusammensetzung nach einem der vorstehenden Ansprüche, **dadurch gekennzeichnet, dass** es sich um ein Pflege- und/oder Make-up-Produkt für die Lippen handelt, insbesondere um einen Lippenstift, einen Lippenkonturstift oder einen Lippenbalsam.

9. Kosmetisches Verfahren zur Pflege und/oder zum Schminken der Lippen, umfassend das Auftragen mindestens einer Zusammensetzung, wie in einem der vorstehenden Ansprüche definiert, auf die Lippen.

## Claims

1. Solid, preferably anhydrous cosmetic composition comprising, in a physiologically acceptable medium at least:
an organopolysiloxane elastomer conveyed in a first oil and an organopolysiloxane elastomer powder distinct from the organopolysiloxane elastomer conveyed in a first oil,
a nonpolar oil distinct from the first oil,
a polar wax,
the total active content of organopolysiloxane elastomer(s) being greater than or equal to 7%, preferably greater than or equal to 10% by weight of the total weight of said composition, and the total wax content being greater than or equal to 5% by weight of the total weight of said composition.

2. Composition according to claim 1, **characterized in that** said organopolysiloxane elastomer powder is coated with silicone resin.

3. Cosmetic composition according to any one of claims 1 or 2, **characterized in that** the active ingredient content of organopolysiloxane elastomer conveyed in an oil ranges from 0.1% to 10%, in particular 1% to 8%, preferably 2% to 6% by weight relative to the total weight of the composition, and the organopolysiloxane elastomer powder content ranges from 1% to 20%, in particular 5% to 15%, preferably 7% to 11% by weight relative to the total weight of the composition.

4. Cosmetic composition according to any of the preceding claims, **characterized in that** the polar wax is selected from the group consisting of candelilla wax, beeswax, carnauba wax, rice bran wax, sunflower wax, jojoba wax and mixtures thereof, preferably candelilla wax.

5. Cosmetic composition according to any of the preceding claims, **characterized in that** it further comprises at least one nonpolar wax.

6. Cosmetic composition according to any of the preceding claims, **characterized in that** it further comprises a polar oil.

7. Cosmetic composition according to any of the preceding claims, **characterized in that** it further comprises coloring materials, preferably pigments.

8. Cosmetic composition according to any of the preceding claims, **characterized in that** it is a lip care and/or makeup product, in particular a lipstick, a lip liner, or a lip balm.

9. Cosmetic method for lip care and/or makeup comprising applying to the lips at least one composition as defined in any of the preceding claims.
